# EUROPÄISCHE PATENTSCHRIFT

(11) **EP 2 001 406 B1**
(45) Veröffentlichungstag und Bekanntmachung des Hinweises auf die Patenterteilung: **24.07.2013**
(21) Anmeldenummer: 07703068.2
(22) Anmeldetag: 26.01.2007
(51) Int. Cl.: A61F 2/14

(54) **VORRICHTUNG ZUR REVERSIBLEN BEFESTIGUNG EINES IMPLANTATS IM AUGE**
DEVICE FOR REVERSIBLY ATTACHING AN IMPLANT IN AN EYE
DISPOSITIF DE FIXATION RÉVERSIBLE D'UN IMPLANT DANS L'OEIL

(30) Priorität: 31.03.2006 DE 102006015113
(43) Veröffentlichungstag der Anmeldung: 17.12.2008
(73) Patentinhaber: Pixium Vision SA, 75012 Paris (FR)
(72) Erfinder: TIEDTKE, Hans-Jürgen, 53227 Bonn (DE); MEYER, Alexander, 51379 Leverkusen-Opladen (DE)
(74) Vertreter: Graf von Stosch, Andreas
(86) Internationale Anmeldenummer: PCT/EP2007/000684
(87) Internationale Veröffentlichungsnummer: WO 2007/118526

(56) Entgegenhaltungen:
- EP-A2- 0 138 564
- WO-A-03/039661
- WO-A-2004/112893
- US-A- 4 586 929
- US-A1- 2004 078 064

## Beschreibung

Die vorliegende Erfindung bezieht sich auf eine Vorrichtung zur reversiblen Befestigung eines Implantats nach dem Oberbegriff des Anspruchs 1 am Auge oder im Auge eines Menschen oder eines Säugetieres. Die vorliegende Erfindung bezieht sich insbesondere auf eine Vorrichtung zur reversiblen Befestigung eines Implantats sowie auf ein Werkzeug zur Handhabung der erfindungsgemäßen Vorrichtung.

Eine häufige Ursache für den teilweisen oder vollständigen Verlust des Sehvermögens ist die Zerstörung der Photorezeptorschicht in der Retina des menschlichen Auges, wonach auftreffende Photonen nicht in entsprechende Reize der Ganglienzellen umgesetzt werden. Bei diesem Krankheitsbild sind die Ganglienzellen nur teilweise betroffen, sodass eine externe Reizung der in der Retina noch vorhandenen Ganglienzellen eine visuelle Wahmehmung erzeugen kann. Auf dieser Basis werden seit einiger Zeit Entwicklungen vorgenommen, welche die Implantation einer Mikrokontaktstruktur mit Stimulations-Elektroden zur Kontaktierung von intakten Ganglienzellen beinhalten.

Es sind Vorrichtungen in Form von passiven oder aktiven Implantaten für die Netzhaut (Retina) des menschlichen Auges bekannt, die zur Behandlung von Patienten vorgesehen sind, deren Sehvermögen teilweise oder vollständig durch Defekte in der Retina verloren gegangen ist. Bei aktiven Implantaten wird prinzipiell das durch eine externe Kamera erfasste Bild in elektrische Signale umgesetzt und über die Stimulations-Elektroden der Mikrokontaktstruktur mittels elektrischer Stimulations-Impulse an die Ganglienzellen der Retina bzw. an den Sehnerv abgegeben, um so das Sehvermögen des erblindeten oder teilweise erblindeten Patienten wiederherzustellen bzw. zu verbessern.

Solche Mikrokontaktstrukturen bestehen im Wesentlichen aus einer Implantatfolie, die elektrisch leitende, stift- oder nadelförmig ausgebildete Kontaktelemente trägt, die gleichmäßig über die Oberfläche der Implantatfolie verteilt sind und über die Ebene der Implantatfolie hervorstehen, um die Ganglienzellen der Retina zu kontaktieren. Um eine gute Funktionalität solcher Implantate zu gewährleisten, müssen die Implantatfolien an der Retina und/oder an der Sklera des Auges zuverlässig fixiert werden.

Die Befestigung eines passiven oder aktiven Implantats im Auge eines Menschen oder eines Säugetieres ist nach dem Stand der Technik per Nagel bzw. Tack oder mittels gezielter Einwucherung erfolgt. Bei der Verwendung eines Nagels wird das Implantat auf die Netzhaut gelegt oder das Implantat wird über den Nagel gelegt und der Nagel dann mit Hilfe einer speziellen Zange in die Netzhaut bzw. Retina und Sklera des Auges gebohrt, wo er sich durch seine Widerhaken in der relativ dicken Sklera dauerhaft verankert.

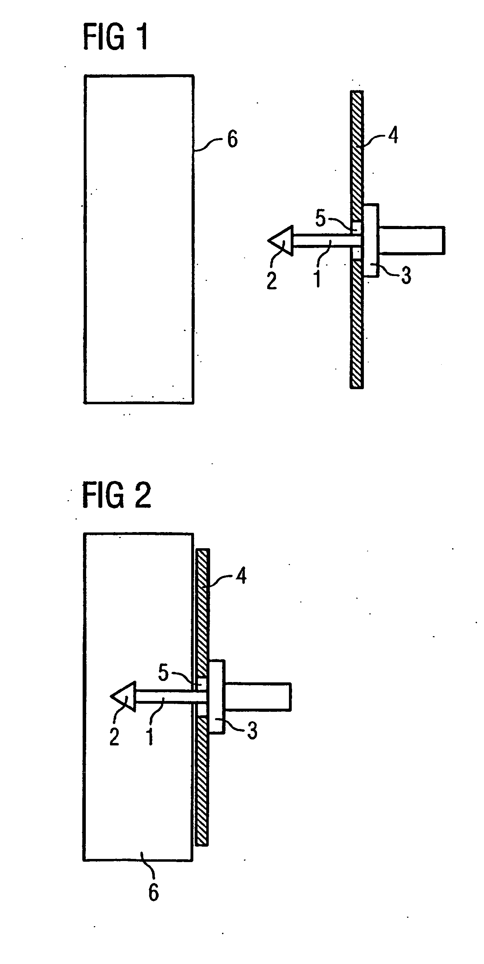

Die Figuren 1 und 2 zeigen den Vorgang nach dem Stand der Technik zum Implantieren eines Implantats im Inneren eines Auges. In Figur 1 ist ein Implantat vor der Befestigung im Auge dargestellt, wobei das Implantat eine Implantatfolie umfasst, die an der Retina/Sklera eines Auges mit Hilfe eines Nagels nach dem Stand der Technik fixiert werden soll. Der Nagel weist an seiner Spitze Widerhaken auf und ist im Bereich seiner Mitte mit einem Kragen versehen, der einen größeren Durchmesser aufweist als der übrige Nagelkörper. Die Implantatfolie weist eine Öffnung auf, die so dimensioniert ist, dass der Nagel hindurchgeführt werden kann, aber einen geringeren Durchmesser aufweist ais der Kragen des Nagels. Zur Befestigung der Implantatfolie an der Retina/Sklera des Auges wird der Nagel so weit in die Öffnung in der Implantatfolie eingeführt, bis der Kragen des Nagels an der Implantatfolie anliegt.

In Figur 2 ist das in Figur 1 gezeigte Implantat nach der Befestigung an der Retina/Sklera des Auges mit Hilfe des Nagels nach dem Stand der Technik dargestellt. In diesem befestigten Zustand ist die Implantatfolie des Implantats an der Retina/Sklera des Auges durch den Nagel fixiert, wobei der Nagel im Gewebe der Retina/Sklera eingebohrt ist und sich durch seine Widerhaken darin verankert. Da der Kragen einen größeren Durchmesser aufweist als die Öffnung in der Implantatfolie, bildet der Kragen des Nagels einen Anschlag, der die Implantatfolie in ihrer Position an der Retina/Sklera des Auges hält.

Bei dieser Befestigung nach dem Stand der Technik kann das Implantat nicht mehr ohne Entfernen des Nagels aus dem Auge entnommen bzw. explantiert werden. Beim Entfernen des Nagels wird jedoch durch dessen Widerhaken ein Teil der Netzhaut irreversibel zerstört, was zu Netzhautablösungen führen kann. Ferner besteht bei der oben beschrieben Befestigungsmethode das Problem, dass Nagel und Implantat zusammen eingesetzt werden müssen und somit im Inneren des Auges gleichzeitig zu hantieren sind, was die Gefahr von Schädigungen der Netzhaut durch unbeabsichtigtes Berühren derselben mit Teilen des Implantats oder des Nagels stark erhöht. Außerdem kann das Implantat leicht durch die scharfen Widerhaken des Nagels beschädigt werden.

Bei einer anderen bekannten Befestigungsmethode, die sich noch in der Entwicklungsphase befindet, erfolgt die Befestigung von Implantaten im Inneren eines Auges mit Klebstoffen, wobei Fibrin-ähnliche Klebstoffe eingesetzt werden. Diese Klebstoffe haben allerdings den Nachteil, dass sie nicht sofort Halt bieten, sondern erst nach relativ langer Zeit aushärten, weshalb das Implantat währenddessen zusätzlich mittels einer anderen Methode zu fixieren ist. Weiterhin können diese Klebstoffe die Gefahr von Zellwucherungen (Proliferation) im Auge erhöhen.

Das Dokument US 2004078064 beschreibt die Präambel des Anspruchs 1.

Eine Aufgabe der vorliegenden Erfindung besteht folglich darin, eine Vorrichtung zur reversiblen Befestigung eines Implantats an einem Auges bereitzustellen, mit der das Implantat auf einfache Weise im Auge oder am Auge befestigt werden kann und bei Bedarf wieder vom Auge gelöst bzw. explantiert werden kann, wobei Beeinträchtigungen oder Verletzungen des Gewebes am Auge auf ein Minimum reduziert werden.

Diese Aufgabe wird nach der vorliegenden Erfindung durch eine Vorrichtung mit den Merkmalen gemäß Anspruch 1 gelöst. Vorteilhafte Weiterbildungen der Erfindung sind in den Unteransprüchen angegeben. Die vorliegende Erfindung löst die oben genannte Aufgabe durch eine Vorrichtung zur Befestigung eines Implantats am Auge eines Menschen oder Säugetieres mittels eines Nagels, der einen im Wesentlichen gestreckten Schaft aufweist, wobei das Implantat eine Implantatfolie zur Kontaktierung von lebendem Gewebe oder Nerven im visuellen System des Auges umfasst, die eine Öffnung aufweist, durch die der Schaft des Nagels zumindest teilweise durchführbar ist, dadurch gekennzeichnet, dass die Vorrichtung ein Halteelement umfasst, das am Schaft des Nagels angeordnet werden kann, so dass das Halteelement am Schaft des Nagels angreift und in einer Befestigungsposition am Nagel fixiert ist.

Durch die vorliegende Erfindung wird eine reversible und möglichst schadenfreie Befestigung eines Implantats am Auge und insbesondere auf der Retina eines Auges ermöglicht. Durch die erfindungsgemäße Vorrichtung wird das Implantat einerseits zuverlässig auf der Netzhaut befestigt, das Implantat kann aber bei Bedarf durch Entfernen des Halteelements bzw. Retainers wieder von der Netzhaut gelöst werden. Das Halteelement lässt sich auf einfache Weise beispielsweise durch eine Pinzette oder ein Skalpell wieder lösen und vom Nagel entfernen. Der Nagel bzw. Tack kann im Augengewebe des Patienten verbleiben und so bei einer erneuten Implantation wieder genutzt werden. Indem der Nagel bei der Entnahme eines Implantats aus dem Auge nicht entfernt werden muss, können auch die Beeinträchtigungen oder Verletzungen des Gewebes am Auge vermieden werden, die ansonsten mit der Entfernung des Nagels verbunden wären. Dies ist insbesondere dann von Vorteil, wenn der Nagel zur Befestigung eines Implantats in der empfindlichen Netzhaut bzw. Retina und/oder Sklera des Auges verankert ist.

Nach einer bevorzugten Ausführungsform der erfindungsgemäßen Vorrichtung weist der Nagel einen Schaft mit einer im Wesentlichen gestreckten Form und einem kreisförmigen Querschnitt auf. Im mittleren Bereich des Schafts ist ein Kragen ausgebildet, der einen größeren Durchmesser aufweist als der Schaft des Nagels. Gemäß einer weiteren bevorzugten Ausführungsform hat der Schaft auf der bezüglich des Kragens zur Spitze des Nagels weisenden Seite (vorderer Teil des Nagels) einen geringeren Durchmesser als auf der anderen Seite des Kragens (hinterer Teil des Nagels). Dadurch lässt sich der vordere Teil des Nagels mit seinem kleineren Durchmesser leichter und bei möglichst geringer Beeinträchtigung des Gewebes im Gewebe platzieren, während der hintere Teil des Nagels mit seinem größeren Durchmesser einen besseren Ansatz für die Anordnung und die Befestigung des Implantats bietet. Der Nagel ist an seiner Spitze mit mindestens einem Widerhaken ausgestattet, so dass der Nagelkörper im Wesentlichen der Form eines Pfeils entspricht, in dessen mittleren Bereich ein tellerförmiger Kragen ausgebildet ist.

Die Implantatfolie des zu befestigenden Implantats weist eine Öffnung auf, die als Loch, Langloch, Einkerbung oder eine andere geeignete Aussparung ausgebildet sein kann. Die Öffnung in der Implantatfolie ist so dimensioniert, dass die Implantatfolie auf den Schaft des Nagels aufgesetzt werden kann, indem der Schaft des Nagels mit seinem hinteren Teil bis zum Kragen durch die Öffnung in der Implantatfolie eingeführt werden kann. Die Öffnung in der Implantatfolie weist jedoch einen geringeren Durchmesser auf als der Kragen des Nagels. Aufgrund dieser Dimensionierung der Öffnung in der Implantatfolie, des Durchmessers vom Schaft des Nagels und des Durchmessers vom Kragen des Nagels, kann die Öffnung in der Implantatfolie so weit auf den hinteren Teil des Schafts vom Nagel geführt werden, bis die Implantatfolie am Kragen des Nagels anliegt.

Zur Befestigung eines Implantats im Inneren eines Auges mittels der Vorrichtung nach der vorliegenden Erfindung wird zunächst der Nagel mit der Spitze und seinem vorderen Teil in das Gewebe bzw. die Retina/Sklera des Auges gebohrt bis der Kragen des Nagels auf dem Gewebe anliegt. Wegen der Widerhaken an der Spitze des Nagels bleibt dieser fest im Gewebe verankert. Mit der erfindungsgemäßen Vorrichtung kann ein Implantat jedoch auch außerhalb des Augapfels, beispielsweise an der Lederhaut des Auges befestigt werden. Nach der Platzierung des Nagels im Gewebe, an der das Implantat fixiert werden soll, kann das Implantat bzw. die Implantatfolie selbst am Nagel angeordnet werden. Dies erfolgt, indem das Implantat auf den der Spitze des Nagels gegenüberliegenden bzw. hinteren Teil des im Gewebe verankerten Nagels aufgesetzt wird, wobei der hintere Teil des Schafts vom Nagel in die Öffnung in der Implantatfolie eingeführt wird, bis die Implantatfolie am Kragen des Nagels anliegt.

Anschließend wird das Halteelement unmittelbar hinter der Implantatfolie auf dem hinteren Teil des Schafts vom Nagel platziert. Die Abmessungen des Halteelements sind so gestaltet, dass das Halteelement nicht durch die Öffnung in der Implantatfolie passt, sondern auf der Rückseite der Implantatfolie anliegt. Das Halteelement wird am hinteren Teil des Schafts vom Nagel auf der vom Gewebe abgewendeten Rückseite der Implantatfolie vorzugsweise unmittelbar hinter der Implantatfolie befestigt. Auf diese Weise ist die Implantatfolie des Implantats an der Retina/Sklera des Auges durch den im Gewebe verankerten Nagel und das am Nagel befestigte Halteelement fixiert.

Das Halteelement hat vorzugsweise eine selbständige Klemmwirkung, so dass es sich nach der Platzierung in der gewünschten Position am Schaft des Nagels selbständig fixiert. Dazu kann das Halteelement beispielsweise als Gummiring ausgeführt sein, der in dem am Schaft des Nagels befestigten Zustand größere Abmessungen aufweist als die Öffnung in der Implantatfolie und damit die Implantatfolie auf dem Schaft des Nagels hält. Wenn der Gummiring im entspannten Zustand einen geringeren Durchmesser aufweist als der hintere Teil des Schafts vom Nagel, entfaltet der Gummiring eine Zugspannung und damit einen Anpressdruck, wenn er auf den Schaft des Nagels aufgezogen wird, so dass er sich im befestigten Zustand nach seiner Platzierung am Schafts des Nagels selbständig fixiert und damit die Implantatfolie in der gewünschten Position hält. Auf diese Weise wird das Implantat bzw. die Implantatfolie so an dem Nagel befestigt, dass es sich nicht durch Augenbewegungen oder Beschleunigungen, die z.B. im Alltag des Implantat-Trägers auftreten, lösen kann.

Das Halteelement kann auch als Klemmfeder ausgebildet sein, das den Schaft des Nagels im befestigten Zustand nicht vollständig umgeben muss, aber zumindest teilweise umschließt. Dieses als Klemmfeder ausgebildete Halteelement ist vorteilhafterweise mit einer Vorspannung versehen, so dass sich das Halteelement mit immanenter Federkraft im befestigten Zustand am Schaft des Nagels fixiert und damit die Implantatfolie in der gewünschten Position hält. Die Ausgestaltung des Halteelements als Gummiring oder Klemmfeder hat ferner den Vorteil, dass das Halteelement durch Dehnung gegen die Federkraft bzw. gegen die Zugkraft bzw. Anpresskraft des Gummis bei Bedarf wieder vom Schaft des Nagels entfernt werden kann, um z.B. das Implantat wieder zu explantieren oder das Halteelement neu zu positionieren.

Weitere Einzelheiten, bevorzugte Ausführungsformen und Vorteile der vorliegenden Erfindung ergeben sich aus der nachfolgenden Beschreibung unter Bezugnahme auf die beigefügten Zeichnungen. Es zeigen:
- Figur 1: die schematische Darstellung eines vorbereitenden Stadiums beim oben beschriebenen Vorgang zum Befestigen eines Implantats mittels einer Befestigungsvorrichtung nach dem Stand der Technik im Inneren eines Auges;
- Figur 2: die schematische Darstellung des in Figur 1 gezeigten Implantats im befestigten Stadium, das mittels der oben beschriebenen Befestigungsvorrichtung nach dem Stand der Technik im Inneren eines Auges befestigt ist;
- Figur 3: die schematische Darstellung eines ersten Stadiums beim Vorgang zum Befestigen eines Implantats im Inneren eines Auges mittels einer Befestigungsvorrichtung gemäß einer bevorzugten Ausführungsform der vorliegenden Erfindung;
- Figur 4: die schematische Darstellung eines zweiten Stadiums beim Vorgang zum Befestigen eines Implantats im Inneren eines Auges mittels einer Befestigungsvorrichtung gemäß einer bevorzugten Ausführungsform der vorliegenden Erfindung;
- Figur 5: die schematische Darstellung eines dritten Stadiums beim Vorgang zum Befestigen eines Implantats im Inneren eines Auges mittels einer Befestigungsvorrichtung gemäß einer bevorzugten Ausführungsform der vorliegenden Erfindung;
- Figur 6: die schematische Darstellung eines vierten Stadiums beim Vorgang zum Befestigen eines Implantats im Inneren eines Auges mittels einer Befestigungsvorrichtung gemäß einer bevorzugten Ausführungsform der vorliegenden Erfindung;
- Figur 7: die schematische Darstellung eines fünften Stadiums beim Vorgang zum Befestigen eines Implantats im Inneren eines Auges mittels einer Befestigungsvorrichtung gemäß einer bevorzugten Ausführungsform der vorliegenden Erfindung;
- Figur 8: die schematische Darstellung eines Implantats, das mittels einer Vorrichtung gemäß einer weiteren bevorzugten Ausführungsform der vorliegenden Erfindung im Inneren eines Auges befestigt wurde;
- Figur 9: die schematische Darstellung eines Werkzeugs zur Handhabung einer Befestigungsvorrichtung gemäß einer bevorzugten Ausführungsform der vorliegenden Erfindung; und die
- Figuren 10 bis 13: zeigen mehrere Darstellungen einer Anzahl von Nägeln in unterschiedlichen Ausführungsformen, wie sie bei einer erfindungsgemäßen Vorrichtung zum Befestigen eines Implantats an einem Auge eingesetzt werden können.

Im folgenden Abschnitt wird eine bevorzugte Ausführungsform der vorliegenden Erfindung anhand einer Vorgehensweise beschrieben, wie die Implantation und Befestigung eines Implantats im Auge mittels der erfindungsgemäßen Vorrichtung unter zu Hilfenahme eines speziellen Werkzeuges erfolgt.

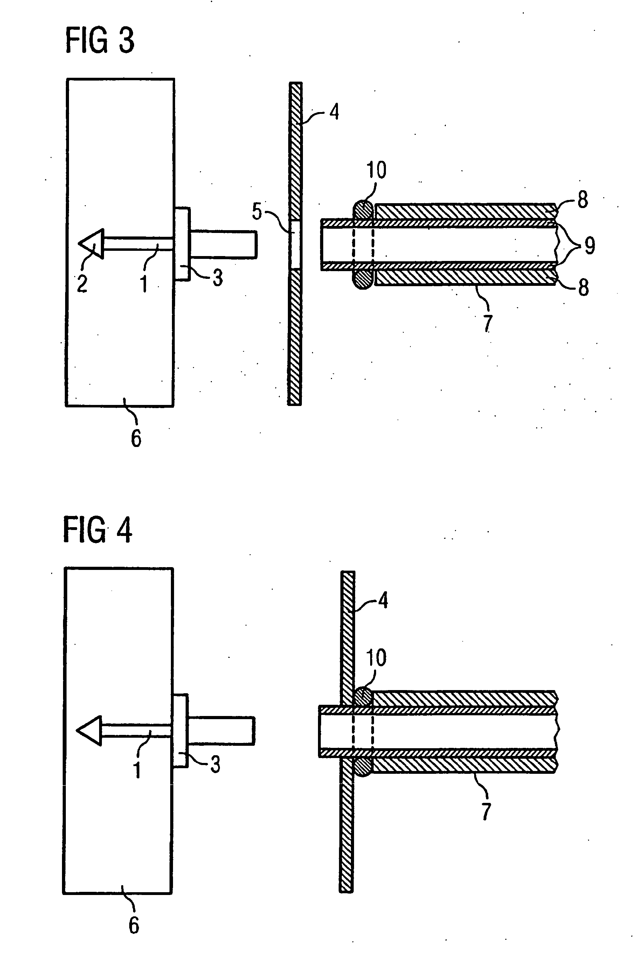

In den Figuren 3 bis 8 zeigen jeweils schematische Darstellungen eines Abschnitts der Retina/Sklera 6 eines behandelten Auges, an der ein Implantat befestigt wird. Bei dem in Figur 3 gezeigten ersten Stadium beim Vorgang zum Befestigen eines Implantats im Inneren eines Auges mittels der erfindungsgemäßen Vorrichtung ist bereits ein Nagel bzw. Tack 1 in der Netzhaut und/oder Sklera 6 des behandelten Auges verankert, indem der Nagel 1 zuvor mit seinem vorderen Teil vollständig in der Retina/Sklera 6 ohne das Implantat eingebracht wurde und mit den Widerhaken an der Spitze in der Retina/Sklera 6 des Auges verankert ist. Das Implantat bzw. die Implantatfolie 4 ist bei dem Vorgang zum Einsetzen und Verankern des Nagels 1 nicht beteiligt. Dies hat den Vorteil, dass sich der Nagel 1 zunächst ohne das Implantat 4 sehr präzise platzieren lässt, da sich das Implantat 4 noch nicht im Auge befindet und nicht zusammen mit dem Nagel 1 in dem beengten Raum des Augeninneren und unter den eingeschränkten Sichtverhältnissen hantiert werden muss.

Bei der in Figur 3 gezeigten Situation befindet sich die Implantatfolie 4 zwischen der Retina/Sklera 6 und dem speziellen Werkzeug 7, das im Wesentlichen aus zwei ineinander geführten und zueinander verschiebbaren Rohren 8 und 9 besteht. Wie in Figur 3 zu erkennen ist, ragt das innere Rohr 9 an der zur Implantatfolie gerichteten Stirnseite um eine definierte Länge über das äußere Rohr 8 hinaus bzw. aus dem äußeren Rohr 8 des Werkzeug 7 heraus. Auf dem überstehenden Teil des inneren Rohres 8 ist ein Halteelement in Form eines Gummirings 10 aus elastischem Material (z.B. Silikon) aufgezogen, der das innere Rohr 9 umgibt und gleichzeitig an der Stimfläche des äußeren Rohres 8 des Werkzeugs 7 anliegt.

Die Implantatfolie 4 weist eine Öffnung 5 auf, deren Durchmesser größer ist als der Durchmesser des inneren Rohres 9, aber kleiner ist als der Durchmesser des äußeren Rohres 8. Bei dem in Figur 4 gezeigten zweiten Stadium des Befestigungsvorgangs ist die Öffnung 5 der Implantatfolie 4 auf das innere Rohr 9 des Werkzeugs 7 aufgesetzt, indem das innerer Rohr in die Öffnung 5 in der Implantatfolie 4 eingeführt wurde. Derart miteinander verbunden werden Werkzeug 7 und Implantatfolie 4 dem in der Retina/Sklera 6 verankerten Nagel 1 angenähert. Bei einer Variante des Vorgang zum Befestigen eines Implantats im Inneren eines Auges mittels der erfindungsgemäßen Vorrichtung wird die Implantatfolie 4 nicht auf das Werkzeug 7 gesetzt, sondern die Implantatfolie 4 wird zuerst auf dem aus der Retina/Sklera 6 hervorstehenden hinteren Teil des Nagels 1 platziert, indem der Schaft des Nagels 1 in die Öffnung 5 der Implantatfolie 4 geführt wird. Anschließend kommt das Werkzeug 7 zum Einsatz, um die Implantatfolie 4 am Nagel 1 zu fixieren, was nachfolgend beschrieben wird.

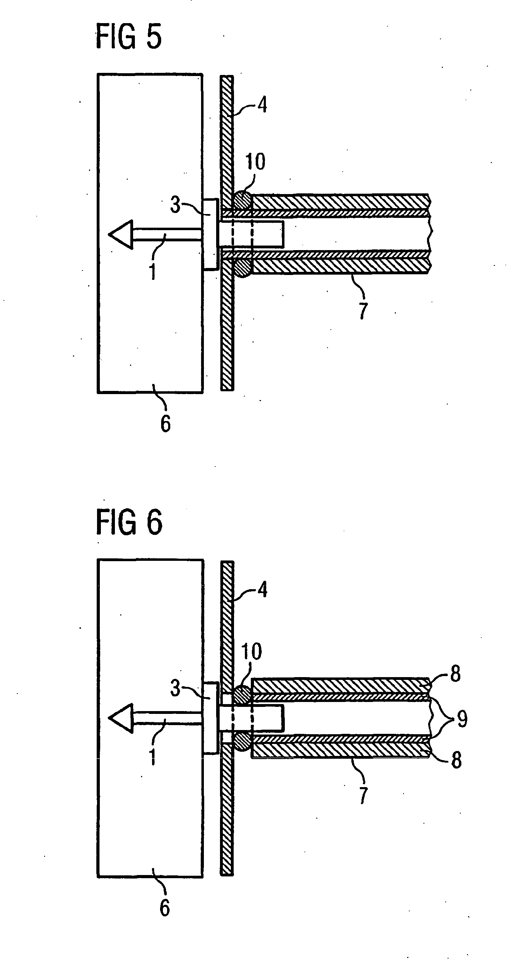

Bei dem in Figur 5 gezeigten dritten Stadium des Befestigungsvorgangs ist das Werkzeug 7 zusammen mit der Implantatfolie 4 auf den hinteren Teil des Nagels 1 aufgesetzt. Der Durchmesser des inneren Rohres 9 ist so dimensioniert, dass er den hinteren Teil des Nagels 1 aufnehmen kann. Auf diese Weise kann das Werkzeug 7 auf den hinteren Teil des Nagels 1 aufgesetzt und auf dem Schaft des Nagels 1 bis zum Anschlag am Kragen 3 des Nagels 1 vorgeschoben werden. Dabei wird das Werkzeug 7 so weit auf den Schaft des Nagels 1 geschoben, bis das innere Rohr 9 des Werkzeugs 7 am Kragen 3 des Nagel 1 anstößt.

Anschließend wird die auf den Schaft des in der Retina/Sklera 6 verankerten Nagels 1 aufgesetzte Implantatfolie 4 fixiert, was mittels des Halteelements bzw. des Gummirings 10 erfolgt. Bei dem in Figur 6 gezeigten vierten Stadium des Befestigungsvorgangs ist das Werkzeug 7 noch auf dem hinteren Teil des Nagels 1 aufgesetzt, aber das innere Rohr 9 ist in das äußere Rohr 8 zurückgezogen, so dass die Stirnflächen der beiden Rohre 8, 9 miteinander fluchten. Beim Zurückziehen des inneren Rohres 9 in das äußere Rohr 8 wird der Gummiring 10 vom äußeren Rohr mit dem größeren Durchmesser in seiner Position gehalten und vom inneren Rohr 9 abgestreift. Dadurch sitzt der Gummiring 10 nun direkt auf dem Schaft des Nagels 1 auf. Aufgrund der Vorspannung des elastischen Materials hat sich der Gummiring 10 um den Schaft des Nagels 1 zusammengezogen.

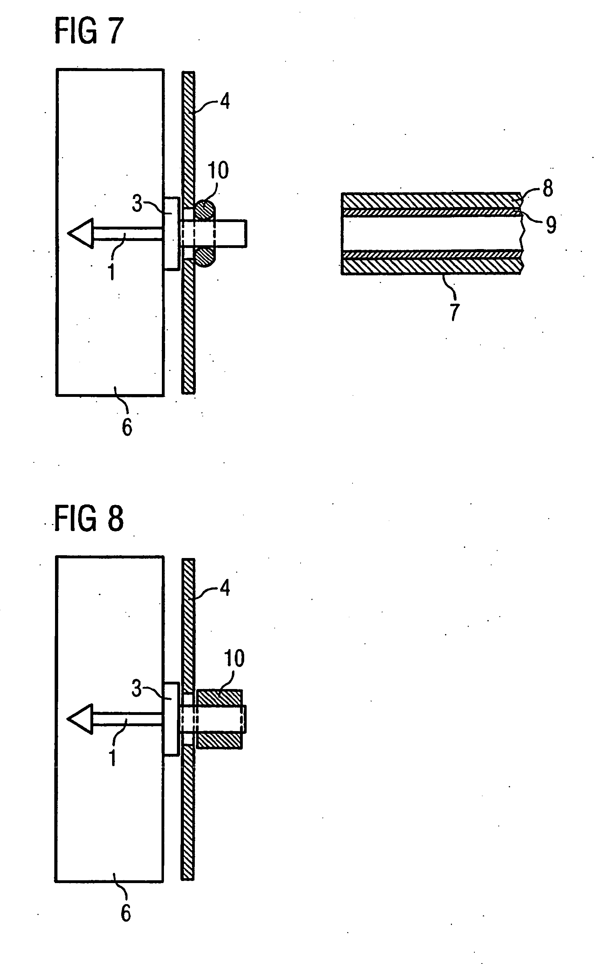

Danach kann das Werkzeug 7 wieder entfernt werden, wie in Figur 7 dargestellt. Der Gummiring 10 entwickelt durch seine Zugkraft bzw. Anpresskraft und der Haftreibung zwischen dem Gummiring 10 und dem Schaft des Nagels 1 eine befestigende Wirkung, wodurch sich der Gummiring 10 am Schaft des Nagels 1 "festhält". Da der Gummiring 10 auch in dem zusammengezogenen Zustand einen größeren Durchmesser hat als die Öffnung 5 in der Implantatfolie 4, ist die Implantatfolie 4 durch den Gummiring 10 in seiner Position am Schaft des Nagels 1 fixiert.

Alternativ kann die Befestigung des Implantats auf dem Schaft des Nagels auch mit Hilfe einer Klammer erfolgen, die den Schaft des Nagels 1 zumindest teilweise umgreift. Ferner kann die Befestigung des Implantats auf dem Schaft des Nagels auch mit einem Splint z.B. aus Polyimid erfolgen, der in einer Vertiefung bzw. Einkerbung im Schaft des Nagels geschoben wird oder mit einer Schraube, die auf ein Gewinde des Nagels gedreht wird.

Figur 8 zeigt die schematische Darstellung eines Implantats, das mittels einer Vorrichtung gemäß einer weiteren bevorzugten Ausführungsform der vorliegenden Erfindung im Inneren eines Auges befestigt wurde ,wobei nur das Endstadium im befestigten Zustand dargestellt ist. Im Unterschied zu der oben beschriebenen Ausführungsform ist bei dieser weiteren Ausführungsform der Gummiring 10 nicht als Torus, sondern als Hülse ausgebildet. Bei dieser Ausgestaltung kann der Gummiring 10 den Schaft des Nagels 1 in der Art einer Ummantelung umschließen. Dadurch ist die Haftreibung zwischen dem Gummiring 10 und dem Schaft des Nagels 1 aufgrund der größeren Kontaktfläche erhöht, was die befestigende Wirkung des Gummirings 10 und damit die Fixierung der Implantatfolie 4 verbessert.

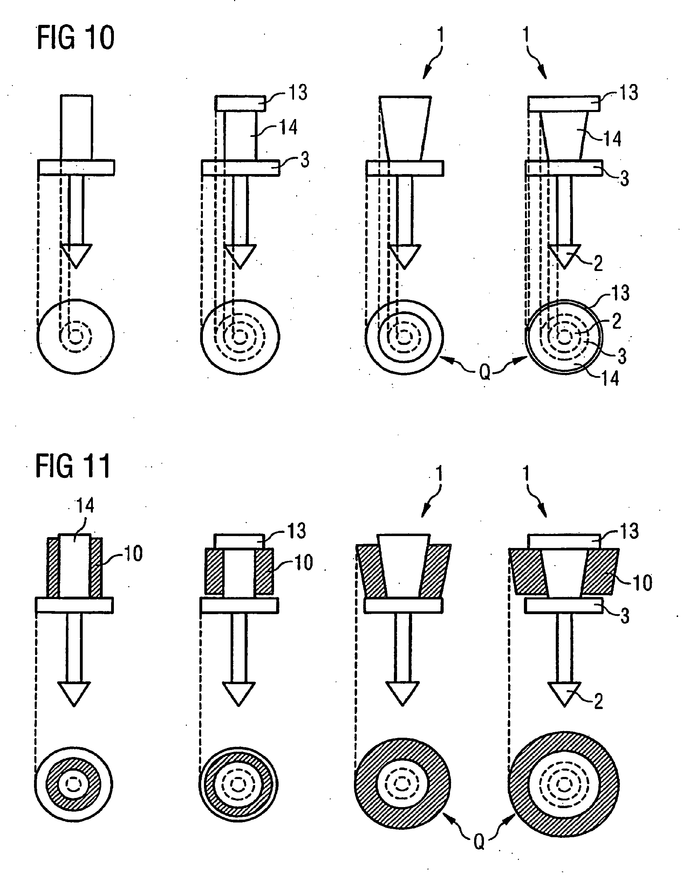

In den Figuren 10 bis 13 sind jeweils mehrere Darstellungen von Nägeln bzw. Tacks in unterschiedlichen Ausführungsformen gezeigt, wie sie bei einer erfindungsgemäßen Vorrichtung zum Befestigen eines Implantats an einem Auge eingesetzt werden können. Dabei sind die Nägel 1 im oberen Teil der Figuren 10 bis 13 jeweils in der Seitenansicht und im unteren Teil der Figuren im Querschnitt Q dargestellt. Während in der Figur 10 die Nägel 1 jeweils ohne ein Halteelement 10 dargestellt sind, zeigen die Figuren 11 bis 13 die Nägel 1 zusammen mit einem daran angeordneten Halteelement 10.

Bei zwei der in den Figuren 10 und 11 dargestellten Ausführungsformen weist der hintere Bereich des Nagels 1 einen konisch ausgebildeten Bereich 14 auf, dessen Durchmesser sich von hinten bis zum Kragen 3 des Nagels 1 verjüngt. Bei anderen Ausführungsformen ist der Nagel 1 jeweils mit einer Hinterschneidung 13 versehen, die einen größeren Durchmesser aufweist als der hintere Bereich des Nagels 1 und auch einen größeren Durchmesser aufweist als der konisch ausgebildete Bereich 14. In den Querschnitten Q der betreffenden Ausführungsformen sind die Durchmesser der einzelnen Elemente des Nagels 1 dargestellt.

In Figur 11 sind die in Figur 10 gezeigten Ausführungsformen der Nägel bzw. Tacks 1 zusammen mit den daran angeordneten Halteelementen 10 dargestellt. Darin lässt sich erkennen, dass die Hinterschneidung 13 einen Anschlag bildet, der eine Verschiebung der Halteelemente 10 verhindert. Bei den Ausführungsformen mit dem konisch ausgebildeten Bereich 14 ist zu erkennen, dass der konische Abschnitt am Schaft des Nagels jeweils mit dem Halteelement 10 zusammenwirkt und dadurch das Halteelement 10 in Richtung zur Spitze 2 des Nagels gedrückt wird, bis das Halteelement 10 am Kragen 3 des Nagels anliegt. Dadurch wird eine Implantatfolie im befestigten Zustand ebenfalls in Richtung der Spitze 2 an den Kragen 3 des Nagels 1 gedrückt.

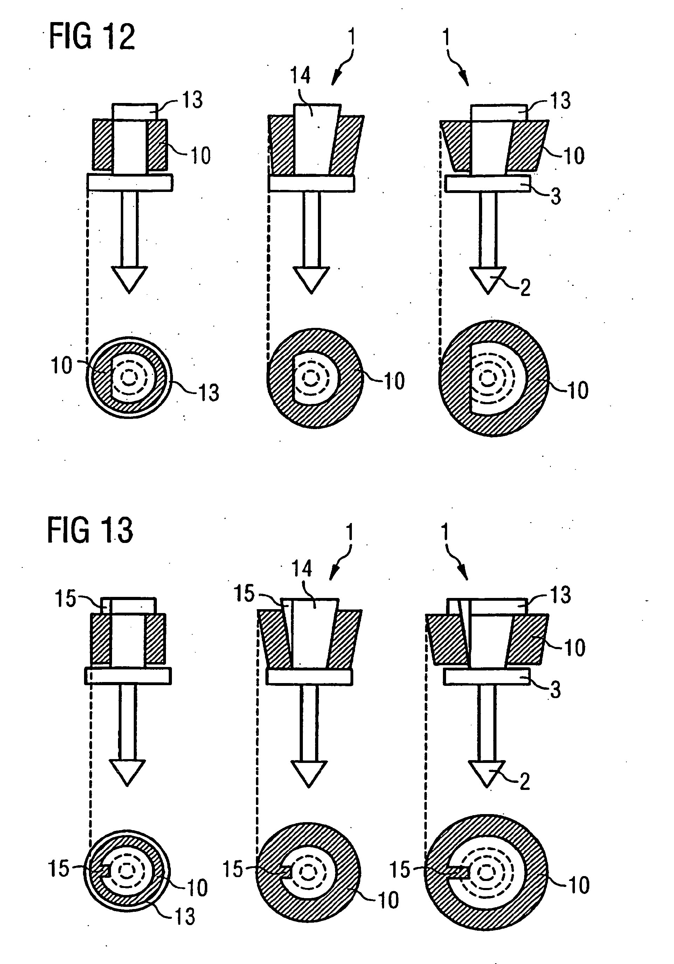

Bei den in Figur 12 dargestellten Ausführungsformen der Nägel bzw. Tacks 1 ist teilweise die Hinterschneidung 13 einseitig abgeflacht, um das Aufziehen des Halteelements 10 auf den Schaft des Nagels 1 zu erleichtern. Zusätzlich oder alternativ kann auch der konische Bereich 14 einseitig abgeflacht sein. Bei den in Figur 13 dargestellten Ausführungsformen der Nägel bzw. Tacks 1 ist die Hinterschneidung 13 und/oder der konische Bereich 14 am Schaft des Nagels 1 mit einer Kerbe 15 versehen, in die eine Nut am inneren Umfang des Halteelements 10 im befestigten Zustand eingreifen kann, um eine Verdrehung des Halteelements 10 am Schaft des Nagels 1 zu verhindern.

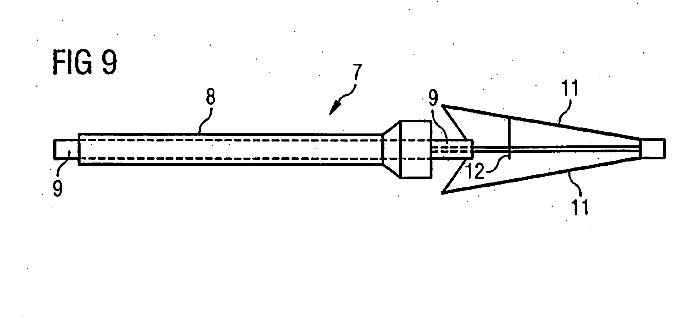

Die Implantation und Befestigung eines Implantats im Auge mittels der erfindungsgemäßen Vorrichtung erfolgt vorzugsweise mit Hilfe eines speziellen Werkzeuges (Retainer Tool). Figur 9 zeigt die schematische Darstellung eines Werkzeugs zur Handhabung einer Befestigungsvorrichtung gemäß einer bevorzugten Ausführungsform der vorliegenden Erfindung. Das Werkzeug 7 besteht in dieser bevorzugten Ausführungsform aus zwei ineinander gesteckten und zueinander beweglichen Rohren 8 und 9, wobei in einer ersten Position das Innenrohr 9 an der Stirnseite teilweise aus dem Außenrohr 8 hervorragt. Da das Innenrohr 9 und das Außenrohr 8 zueinander verschiebbar sind, lässt sich das Innenrohr 9 gegenüber dem Außenrohr 8 zurückziehen und so in eine zweite Position bringen, bei der das Innenrohr 9 an der Stirnseite nicht mehr aus dem Außenrohr 8 hervorragt, sondern mit diesem bündig abschließt oder das Innenrohr 9 in das Außenrohr 8 zurückgezogen ist.

Anstelle des Innenrohrs 9 und des Außenrohrs 8 können auch andere beliebig geformte Hohlkörper verwendet werden. Wichtig ist dabei, dass die verwendeten Hohlkörper ineinander geführt werden können und relativ zueinander beweglich sind. Als weiteres Merkmal sollte der innere Hohlkörper 9 zumindest an seiner operativen Stirnseite, mit der das Halteelement 10 (und die Implantatfolie 4) auf dem Nagel 1 platziert wird, einen hohlen Abschnitt aufweisen, damit der innere Hohlkörper 9 mit seiner Stirnseite auf dem Nagel 1 aufgesetzt werden kann. Ferner ist es von Vorteil, wenn der innere Hohlkörper 9 zumindest an seiner Stirnseite runde Außenabmessungen aufweist, damit das unter Vorspannung stehende Halteelement 10 leicht darauf aufgezogen und wieder davon abgestreift werden kann, um es auf dem Schaft des Nagels 1 zu platzieren, wie oben beschrieben.

Auf der von der Stirnseite der Rohre 8 und 9 gegenüberliegenden Seite ist das Werkzeug 7 mit einer Betätigungsmechanik ausgestattet, mit der die relative Bewegung des Innenrohrs 9 gegenüber dem Außenrohr 8 bewirkt werden kann. Bei der in Figur 9 dargestellten Ausführungsform umfasst die Betätigungsmechanik des Werkzeugs 7 zwei Griffschenkel 11, die in einem gemeinsamen Endpunkt gelenkig gelagert sind und an ihrem freien Ende jeweils mit dem Innenrohr 9 verbunden sind. Wenn die Griffschenkel 11 zusammengedrückt werden, bewirkt die Betätigungsmechanik, dass das Innerohr 9 aus dem Außenrohr 8 in Richtung des Griffes 11 herausgezogen wird, d.h. dass das Innerohr 9 an der dem Griff 11 gegenüberliegenden Stirnseite in das Außenrohr 8 zurückgezogen wird. Die Betätigungsmechanik des Werkzeugs 7 ist ferner mit einem mechanischen Anschlag 12 ausgestattet, der die Bewegung der Griffschenkel 11 begrenzt. Dieser mechanische Anschlag 12 ist vorzugsweise so ausgebildet, dass die Griffschenkel 11 so weit zusammengedrückt werden können, bis das Innerohr 9 an der Stirnseite vollständig in das Außenrohr 8 zurückgezogen ist. Das Innenrohr 9 ist dabei auf einer Achse geführt, die sich aus dem Innenrohr 9 bis zum gemeinsamen Endpunkt der Griffschenkel 11 erstreckt.

Besonders vorteilhaft ist es, wenn die Griffschenkel 11 durch eine Vorspannung in der geöffneten Position gehalten werden, in der das Innerohr 9 an der Stirnseite aus dem Außenrohr 8 herausragt. Dadurch kann das Werkzeug 7 zusammen mit dem auf dem hervorstehenden Teil des Innenrohrs 9 aufgezogenen Halteelement 10 (und der aufgesetzten Implantatfolie 4) am verankerten Nagel 1 in Position gebracht werden, während die Griffschenkel 11 durch die Vorspannung (beispielsweise einer Feder) zuverlässig in der geöffneten Position bleiben. Erst wenn das Werkzeug 7 zusammen mit dem Halteelement 10 und der aufgesetzten Implantatfolie 4 am verankerten Nagel 1 in die gewünschte Position gebracht worden sind, können die Griffschenkel 11 des Werkzeugs 7 gegen die Vorspannung zusammengedrückt und damit die Betätigungsmechanik betätigt werden, die an der Stirnseite des Werkzeugs 7 das Zurückziehen des Innerohrs 9 in das Außenrohr 8 bewirkt. Dadurch wird das Halteelement 10 auf dem Schaft des Nagels 1 abgesetzt und die Implantatfolie 4 in der gewünschten Position fixiert, wie oben beschrieben.

Bei einem Implantat, das mit der erfindungsgemäßen Vorrichtung befestigt ist, kann eine eventuell erforderliche Explantation in der Weise geschehen, indem zunächst das Halteelement 10 vom Schaft des Nagels entfernt wird. Dies kann durch Abziehen z.B. mit einer Pinzette geschehen. Alternativ kann das Halteelement z.B. mit einem Skalpell oder einem anderen Werkzeug längs des Nagelschaftes aufgeschnitten und entfernt werden. Danach kann das Implantat 4 vom Schaft des Nagels 1 abgehoben werden, wobei der in der Retina/Sklera verankerte Nagel 1 im Auge des Implantat-Trägers verbleibt und für eine spätere Re-Implantation vorteilhaft genutzt werden kann.

**Liste der Bezugszeichen**

| | |
|---|---|
| 1 | Nagel |
| 2 | Spitze des Nagels |
| 3 | Kragen des Nagels |
| 4 | Implantat bzw. Implantatfolie |
| 5 | Öffnung in der Implantatfolie |
| 6 | Retina/Sklera des Auges |
| 7 | Werkzeug |
| 8 | äußeres Rohr des Werkzeugs |
| 9 | inneres Rohr des Werkzeugs |
| 10 | Halteelement bzw. Gummiring |
| 11 | Griff am Werkzeug |
| 12 | Anschlag am Werkzeug |
| 13 | Hinterschneidung am Nagel |
| 14 | konischer Abschnitt am Schaft des Nagels |
| 15 | Kerbe in der Hinterschneidung und/oder im Schaft des Nagels |
| Q | Querschnitt vom Nagel |

## Patentansprüche

1. Vorrichtung zur Befestigung eines Implantats am Auge eines Menschen oder Säugetieres mittels eines Nagels (1), der einen im Wesentlichen gestreckten Schaft aufweist, wobei das Implantat eine Implantatfolie (4) zur Kontaktierung von lebendem Gewebe oder Nerven im visuellen System des Auges umfasst und die Implantatfolie (4) eine Öffnung (5) aufweist,
wobei die Vorrichtung ein Halteelement (10) umfasst, das am Schaft des Nagels (1) angeordnet werden kann, so dass das Halteelement (10) am Schaft des Nagels (1) angreift und in einer Befestigungsposition am Nagel (1) fixiert ist,
**dadurch gekennzeichnet, dass** die Abmessungen eines hinteren Teils des Schafts des Nagels (1) so gestaltet sind, dass der hintere Teil des Schafts des Nagels (1) durch die Öffnung (5) der Implantatfolie (4) durchführbar ist, wobei im befestigten Zustand das Halteelement (10) hinter der Implantatfolie (4) auf dem hinteren Teil des Schafts vom Nagel (1) positioniert ist.

2. Vorrichtung nach Anspruch 1, wobei das am Schaft des Nagels (1) angeordnete Halteelement (10) den Schaft des Nagels (1) zumindest teilweise umschließt und mit diesem mechanisch zusammenwirkt, um eine auf den Schaft des Nagels (1) aufgesetzte Implantatfolie (4) in einer Befestigungsposition am Nagel (1) zu fixieren.

3. Vorrichtung nach einem der Ansprüche 1 oder 2, wobei im mittleren Bereich des Schafts vom Nagel (1) ein Kragen (3) ausgebildet ist, der einen größeren Durchmesser aufweist als der Schaft des Nagels (1).

4. Vorrichtung nach Anspruch 3, wobei der Schaft des Nagels (1) auf der bezüglich des Kragens (3) zur Spitze (2) des Nagels (1) weisenden Seite einen geringeren Durchmesser aufweist als auf der anderen Seite des Kragens (3).

5. Vorrichtung nach einem der vorangehenden Ansprüche, wobei der Schaft des Nagels (1) mindestens einen konischen Abschnitt (14) in dem Bereich aufweist, in dem das Halteelement (10) im befestigten Zustand angeordnet wird; und/oder
wobei der Schaft des Nagels (1) mindestens eine Hinterschneidung (13) aufweist, der den Bereich begrenzt, in dem das Halteelement (10) im befestigten Zustand angeordnet wird; und/oder
wobei der Schaft des Nagels (1) mindestens eine Vertiefung oder Kerbe (15) aufweist, in die das Halteelement (10) im befestigten Zustand zumindest teilweise eingreift; und/oder
wobei der Nagel (1) an seiner Spitze (2) mit mindestens einem Widerhaken ausgestattet ist, mit dem sich der Nagel (1) im befestigten Zustand im Gewebe verankert.

6. Vorrichtung nach einem der vorangehenden Ansprüche, wobei die Abmessungen des Halteelements (10) so gestaltet sind, dass das Halteelement (10) im befestigten Zustand nicht durch die Öffnung (5) in der Implantatfolie (4) passt, sondern zumindest teilweise auf der vom Gewebe abgewendeten Rückseite der Implantatfolie (4) anliegt.

7. Vorrichtung nach einem der vorangehenden Ansprüche, wobei das Halteelement (10) aus dem befestigten Zustand zur Lösung der Befestigung des Implantats durch Dehnung oder Öffnung des Halteelements (10) vom Schaft des Nagels (1) lösbar ist.

8. Vorrichtung nach einem der vorangehenden Ansprüche, wobei im befestigten Zustand das Halteelement (10) unmittelbar hinter der Implantatfolie (4) auf dem hinteren Teil des Schafts vom Nagel (1) positioniert ist.

9. Vorrichtung nach einem der vorangehenden Ansprüche, wobei das Halteelement (10) eine selbständige Klemmwirkung aufweist, so dass es sich in seiner Platzierung am Schafts des Nagels (1) selbständig fixiert; und/oder
wobei das Halteelement (10) bevorzugt als Gummiring ausgebildet ist, der im befestigten Zustand größere Abmessungen aufweist als die Öffnung (5) in der Implantatfolie (4); und/oder
wobei das Halteelement (10) bevorzugt als Klemmfeder ausgebildet ist, die vorzugsweise eine Vorspannung aufweist, so dass sich das Halteelement (10) im befestigten Zustand aufgrund seiner Vorspannung am Schaft des Nagels (1) selbständig fixiert.

10. Vorrichtung nach einem der vorangehenden Ansprüche, wobei das Halteelement (10) konische Abmessungen aufweist, die mit dem konischen Abschnitt (14) am Schaft des Nagels (1) zusammenwirken, um im befestigten Zustand einen Druck in Richtung der Implantatfolie (4) auszuüben.

11. Werkzeug (7) zum Handhaben einer Vorrichtung gemäß einem der vorangehenden Ansprüche, mit dessen Hilfe ein Halteelement (10) auf dem Schaft eines Nagels (1) platzierbar ist, um ein Implantat (4) am Auge eines Menschen oder Säugetieres zu befestigen, wobei das Implantat eine Implantatfolie (4) zur Kontaktierung von lebendem Gewebe oder Nerven im visuellen System des Auges umfasst, die eine Öffnung (5) aufweist, durch die der Schaft des Nagels (1) zumindest teilweise durchführbar ist,
**dadurch gekennzeichnet,**
**dass** das Werkzeug (7) zwei ineinander geführte Hohlkörper (8, 9), insbesondere ein Innenrohr (9) und ein Außenrohr (8) aufweist, die in Richtung ihrer Längsachsen zwischen einer ersten Position und einer zweiten Position relativ zueinander beweglich sind, und so dimensioniert ist, dass es auf den hinteren Teil des Schafts des Nagels geschoben werden kann.

12. Werkzeug (7) nach Anspruch 11, wobei in der ersten Position das Innenrohr (9) an der Stirnseite zumindest teilweise aus dem Außenrohr (8) hervorragt und in der zweiten Position das Innenrohr (9) weitgehend in das Außenrohr (8) zurückgezogen ist; und/oder
wobei in der zweiten Position das Innenrohr (9) an der Stirnseite mit dem Außenrohr (8) bündig abschließt oder das Innenrohr (9) in das Außenrohr (8) zurückgezogen ist.

13. Werkzeug (7) nach Anspruch 11 oder 12, wobei auf der von der Stirnseite gegenüberliegenden Seite des Werkzeugs (7) eine Betätigungsmechanik vorgesehen ist, mittels der die relative Bewegung des Innenrohrs (9) gegenüber dem Außenrohr (8) bewirkt werden kann; und/oder
wobei die Betätigungsmechanik vorzugsweise zwei Griffschenkel (11) umfasst, die in einem gemeinsamen Endpunkt gelenkig gelagert sind und an ihrem freien Ende jeweils mit dem Innenrohr (9) verbunden sind.

14. Werkzeug (7) nach Anspruch 13, wobei ein Zusammendrücken der Griffschenkel (11) eine Verschiebung des Innerohrs (9) gegenüber dem Außenrohr (8) bewirkt, so dass Innerohr (9) an der Stirnseite in das Außenrohr (8) zurückgezogen wird; und/oder
wobei die Griffschenkel (11) durch eine Vorspannung in einer geöffneten Position gehalten werden, in der das Innerohr (9) an der Stirnseite aus dem Außenrohr (8) herausragt.

15. Werkzeug (7) nach einem der Ansprüche 11 bis 14, wobei ein mechanischer Anschlag (12) vorgesehen ist, der die Bewegung der Betätigungsmechanik und/oder der Griffschenkel (11) begrenzt;
wobei der mechanische Anschlag (12) vorzugsweise so ausgebildet ist, dass die Griffschenkel (11) so weit zusammengedrückt werden können, bis das Innerohr (9) an der Stirnseite vollständig in das Außenrohr (8) zurückgezogen ist.

16. Werkzeug (7) nach einem der Ansprüche 13 bis 15, wobei das Innenrohr (9) bei der relativen Bewegung gegenüber dem Außenrohr (8) auf einer Achse geführt ist, die sich aus dem Innenrohr (9) bis zum gemeinsamen Endpunkt der Griffschenkel (11) erstreckt.

## Claims

1. Device for attaching an implant to the eye of a human or mammal by means of a pin (1) which comprises a substantially elongated shaft, wherein the implant comprises an implant film (4) for contacting living tissue or nerves in the visual system of the eye, and wherein the implant film (4) comprises an opening (5), wherein the device comprises a holding element (10) that can be arranged on the shaft of the pin (1), so that the holding element (10) engages on the shaft of the pin (1) and is fixed in an attachment position on the pin (1), **characterized in that** the dimensions of a rear portion of the shaft of the pin (1) are such that the rear portion of the shaft of the pin (1) can pass through the opening (5) in the implant film (4),
wherein, in the attached state, the holding element (10) is positioned behind the implant film (4) on the rear portion of the shaft of the pin (1).

2. Device according to claim 1, wherein the holding element (10) arranged on the shaft of the pin (1) at least partially surrounds the shaft of the pin (1) and co-operates mechanically with the latter to fix an implant film (4) placed on the shaft of the pin (1) in an attachment position on the pin (1).

3. Device according to claim 1 or 2, wherein a collar (3) is formed in the middle region of the shaft of the pin (1), the collar having a larger diameter than the shaft of the pin (1).

4. Device according to claim 3, wherein the shaft of the pin (1) of the side with respect to the collar (2) pointing towards the tip (2) of the pin (1) has a smaller diameter than on the other side of the collar (3).

5. Device according to any one of the preceding claims, wherein the shaft of the pin (1) has at least one conical section (14) in the region in which the holding element (10) is arranged in the attached state; and/or
wherein the shaft of the pin (1) has at least one under-cut (13), which limits the region in which the holding element (10) is arranged in the attached state; and/or
wherein the shaft of the pin (1) has at least one depression or notch (15), in which the holding element (10) at least partially engages in the attached state; and/or
wherein the pin (1) is provided at its tip (2) with at least one barb, with which the pin (1) is anchored in the tissue in the attached state.

6. Device according to any one of the preceding claims, wherein the dimensions of the holding element (10) are such that the holding element (10) in the attached state does not pass through the opening (5) in the implant film (4), but abuts at least partially on the rear side of the implant film (4) facing away from the tissue.

7. Device according to any one of the preceding claims, wherein the holding element (10) is releasable from the shaft of the pin (1) from the attached state in order to release the attachment of the implant by expanding or opening the holding element (10).

8. Device according to any one of the preceding claims, wherein, in the attached state, the holding element (10) is positioned immediately behind the implant film (4) on the rear portion of the shaft of the pin (1).

9. Device according to any one of the preceding claims, wherein the holding element (10) has an independent clamping action so that it is independently fixed in its position on the shaft of the pin (1); and/or
wherein the holding element (10) is preferably formed as a rubber ring, which in the attached state is of larger size than the opening (5) in the implant film (4); and/or
wherein the holding element (10) is preferably formed as a clamping spring, which preferably has a pre-tensioning so that the holding element (10) in the attached state, on account of its pre-tensioning, is fixed on the shaft of the pin by itself.

10. Device according to any one of the preceding claims, wherein the holding element (10) has conical dimensions that co-operate with the conical section (14) on the shaft of the pin (1), in order to exert a pressure in the direction of the implant film (4) in the attached state.

11. Tool (7) for manipulating a device according to any one of the preceding claims, with the aid of which a holding element (10) can be placed on the shaft of a pin (1) in order to attach an implant (4) to the eye of a human or mammal, wherein the implant comprises an implant film (4) for contacting living tissue or nerves in the visual system of the eye, the implant film (4) comprising an opening (5) through which the shaft of the pin (1) can at least partially pass,
**characterized in that**
the tool (7) comprising two hollow bodies (8, 9) guided within one another, in particular an inner tube (9) and an outer tube (8), which are movable relative to one another in the direction of their longitudinal axes between a first position and a second position, and which are dimensioned such that they can be slid over the rear portion of the shaft of the pin.

12. Tool (7) according to claim 11, wherein in the first position the front side of the inner tube (9) projects at least partly from the outer tube (8), and in the second position the inner tube (9) is substantially retracted into the outer tube (8); and/or
wherein in the second position the front side of the inner tube (9) is flush with the outer tube (8), or the inner tube (9) is refracted into the outer tube (8).

13. Tool (7) according to claim 11 or 12, wherein an actuating mechanism is provided on the side of the tool (7) opposite the front side, by means of which the relative movement of the inner tube (9) with respect to the outer tube (8) can be implemented; and/or
wherein the actuating mechanism preferably comprises two gripping legs (11) that are supported in an articulated way at a common end point and are respectively connected at their free end to the inner tube (9).

14. Tool (7) according to claim 13, wherein squeezing the gripping legs (11) produces a displacement of the inner tube (9) with respect to the outer tube (8), so that the front side of the inner tube (9) is retracted into the outer tube (8); and/or
wherein the gripping legs (11) are held by a pre-tensioning in an open position, in which the front side of the inner tube (9) projects from the outer tube (8).

15. Tool (7) according to any one of claims 11-14, wherein a mechanical stop (12) is provided that limits the movement of the actuating mechanism and/or of the gripping legs (11);
wherein the mechanical stop (12) is preferably designed so that the gripping legs (11) can be squeezed until the front side of the inner tube (9) is completely retracted into the outer tube (8).

16. Tool (7) according to any one of claims 13-15, wherein the inner tube (9) in its relative movement with respect to the outer tube (8) is guided on an axis that extends from the inner tube (9) as far as the common end point of the gripping legs (11).

## Revendications

1. Dispositif de fixation d'un implant dans l'oeil d'un être humain ou d'un mammifère au moyen d'une broche (1) qui présente une tige substantiellement rectiligne, l'implant comprenant une feuille d'implant (4) pour la mise en contact de tissus vivants ou de nerfs dans le système visuel de l'oeil et la feuille d'implant (4) présentant une ouverture (5),
le dispositif comprenant un élément de retenue (10) qui peut être disposé sur la tige de la broche (1) de sorte que l'élément de retenue (10) intervienne sur la tige de la broche (1) et soit fixé à la broche (1) dans une position de fixation,
**caractérisé en ce que** les dimensions d'une partie arrière de la tige de la broche (1) sont telles que la partie arrière de la tige de la broche (1) peut être insérée par l'ouverture (5) de la feuille d'implant (4), l'élément de retenue (10) étant placé à l'état fixé à l'arrière de la feuille d'implant (4) sur la partie arrière de la tige de la broche (1).

2. Dispositif selon la revendication 1, dans lequel l'élément de retenue (10) disposé sur la tige de la broche (1) entoure au moins partiellement la tige de la broche (1) et coopère mécaniquement avec celle-ci pour fixer une feuille d'implant (4) placée sur la tige de la broche (1) dans une position de fixation sur la broche (1).

3. Dispositif selon l'une des revendications 1 ou 2, dans lequel, un collet (3) qui présente un diamètre supérieur à celui de la tige de la broche (1) est formé dans la zone médiane de la tige de la broche (1).

4. Dispositif selon la revendication 3, dans lequel la tige de broche (1) présente sur le côté tourné vers la pointe (2) de la broche (1) par rapport au collet (3) un diamètre inférieur à celui de l'autre côté du collet (3).

5. Dispositif selon l'une des revendications précédentes, dans lequel la tige de la broche (1) présente au moins un segment conique (14) dans la zone dans laquelle l'élément de retenue (10) est disposé à l'état fixé ; et/ou
dans lequel la tige de la broche (1) présente au moins une contre-dépouille (13) qui est adjacente à la zone dans laquelle l'élément de retenue (10) est disposé à l'état fixé; et/ou
dans lequel la tige de la broche (1) présente au moins une cavité ou une rainure(15) dans lequel l'élément de retenue (10) est au moins partiellement en prise à l'état fixé ; et/ou
dans lequel la broche (1) est dotée à sa pointe (2) d'au moins un crochet avec lequel la broche (1) s'ancre dans le tissu à l'état fixé.

6. Dispositif selon l'une des revendications précédentes, dans lequel les dimensions de l'élément de retenue (10) sont telles que l'élément de retenue (10) ne passe pas, à l'état fixé, par l'ouverture (5) dans la feuille d'implant (4) mais repose au moins partiellement sur le verso de la feuille d'implant (4) à l'opposé du tissu.

7. Dispositif selon l'une des revendications précédentes, dans lequel l'élément de retenue (10) peut être libéré de l'état fixé pour défaire la fixation de l'implant par dilatation ou ouverture de l'élément de retenue (10) de la tige de la broche (1).

8. Dispositif selon l'une des revendications précédentes, dans lequel, à l'état fixé, l'élément de retenue (10) est placé immédiatement à l'arrière de la feuille d'implant (4) sur la partie arrière de la tige de la broche (1).

9. Dispositif selon l'une des revendications précédentes, dans lequel l'élément de retenue (10) présente une action de serrage automatique de sorte qu'il se fixe automatiquement dans sa position sur la tige de la broche (1) ; et/ou dans lequel l'élément de retenue (10) est formé de préférence comme un anneau en caoutchouc qui présente à l'état fixé des dimensions supérieures à celles de l'ouverture (5) dans la feuille d'implant (4) ; et/ou
dans lequel l'élément de retenue (10) est formé de préférence comme un ressort de serrage qui présente de préférence une précontrainte de sorte que l'élément de retenue (10) se fixe automatiquement à l'état fixé sur la tige de la broche (1) en raison de sa précontrainte.

10. Dispositif selon l'une des revendications précédentes, dans lequel l'élément de retenue (10) présente des dimensions coniques qui coopèrent avec le segment conique (14) sur la tige de la broche (1) pour exercer à l'état fixé une pression dans la direction de la feuille d'implant (4).

11. Outil (7) pour manipuler un dispositif selon l'une des revendications précédentes, avec l'aide duquel un élément de retenue (10) peut être placé sur la tige d'une broche (1) pour fixer un implant (4) sur l'oeil d'un être humain ou d'un mammifère, l'implant comprenant une feuille d'implant (4) pour la mise en contact de tissus vivants ou de nerfs dans le système visuel de l'oeil, qui présente une ouverture (5) par laquelle la tige de la broche (1) peut être insérée au moins partiellement,
**caractérisé en ce que**
l'outil (7) présente deux corps creux (8, 9) introduits l'un dans l'autre, en particulier, un tube interne (9) et un tube externe (8) qui sont mobiles l'un par rapport à l'autre, dans la direction de leurs axes longitudinaux entre une première position et une deuxième position, et sont dimensionnés de sorte qu'il puisse être poussé sur la partie arrière de la tige de broche.

12. Outil (7) selon la revendication 11, dans lequel, dans la première position, le tube interne (9) dépasse sur le côté avant au moins partiellement du tube externe (8) et dans la deuxième position, le tube interne (9) est largement retiré dans le tube externe (8) ; et/ou
dans la deuxième position, le tube interne (9) affleure sur le côté avant par rapport au tube externe (8) ou le tube interne (9) est retiré dans le tube externe (8).

13. Outil (7) selon la revendication 11 ou 12, dans lequel, sur le côté opposé à la face avant de l'outil (7), une mécanique d'actionnement est prévue, au moyen de laquelle le mouvement relatif du tube interne (9) peut être actionné par rapport au tube externe (8) ; et/ou
dans lequel la mécanique d'actionnement comprend de préférence deux bras de préhension (11) qui reposent de façon articulée dans un point terminal commun et sont reliés à l'une de leurs extrémités libres, respectivement, au tube interne (9).

14. Outil (7) selon la revendication 13, dans lequel une compression des bras de préhension (11) entraîne un décalage du tube interne (9) par rapport au tube externe (8) de sorte que le tube interne (9) soit retiré sur le côté avant dans le tube externe (8) ; et/ou
dans lequel les bras de préhension (11) sont maintenus par une précontrainte dans une position ouverte dans laquelle le tube interne (9) dépasse sur le côté avant du tube externe (8).

15. Outil (7) selon l'une des revendications 11 à 14, dans lequel une butée mécanique (12) est prévue, qui limite le mouvement de la mécanique d'actionnement et/ou des bras de préhension (11),
la butée mécanique (12) étant conçue de préférence de sorte que les bras de préhensions (11) puissent être comprimés jusqu'à ce que le tube interne (9) sur le côté avant soit complètement retiré dans le tube externe (8).

16. Outil (7) selon l'une des revendications 13 à 15, dans lequel le tube interne (9) est conduit lors du mouvement relatif par rapport au tube externe (8) sur un axe qui s'étend du tube interne (9) au point terminal commun des bras de préhensions (11).
